# EUROPEAN PATENT SPECIFICATION

(11) **EP 1 404 849 B1**
(45) Date of publication and mention of the grant of the patent: **29.12.2010**
(21) Application number: 02738967.5
(22) Date of filing: 30.05.2002
(51) Int. Cl.: C12N 15/82, A01H 5/00, C12N 5/10

(54) **MODIFICATION OF PLANT GENOMES BY INDUCIBLE SITE-SPECIFIC RECOMBINATION OF TRANSGENES**
VERÄNDERUNG DES PFLANZEN GENOMS DURCH INDUZIERBAREN, ORTSPEZIFISCHEN REKOMBINATION VON TRANSGENEN
MODIFICATION DU GENOME DE VEGETAUX PAR UNE RECOMBINATION DIRIGEE ET INDUCTIBLE DES TRANSGENES

(30) Priority: 31.05.2001 EP 01202078
(43) Date of publication of application: 07.04.2004
(73) Proprietor: Stichting Dienst Landbouwkundig Onderzoek, 6701 BH Wageningen (NL)
(72) Inventor: ROUWENDAL, Gerard, Johan, Adolph, NL-6666 CE Heteren (NL)
(74) Representative: Prins, Adrianus Willem
(86) International application number: PCT/NL2002/000349
(87) International publication number: WO 2002/097102

(56) References cited:
- EP-A- 0 911 412
- WO-A-01/21768
- WO-A-01/36595
- WO-A-01/38508
- WO-A-01/40492
- WO-A-01/59086
- WO-A-95/00555
- WO-A-97/37012
- US-A- 5 358 866
- ZUO J ET AL: "CHEMICAL-REGULATED, SITE-SPECIFIC DNA EXCISION IN TRANSGENIC PLANTS" NATURE BIOTECHNOLOGY, NATURE PUBLISHING, US, vol. 19, no. 2, February 2001 (2001-02), pages 157-161, XP001022101 ISSN: 1087-0156 cited in the application
- SUGITA KOICHI ET AL: "A transformation vector for the production of marker-free transgenic plants containing a single copy transgene at high frequency" PLANT JOURNAL, BLACKWELL SCIENTIFIC PUBLICATIONS, OXFORD, GB, vol. 22, no. 5, June 2000 (2000-06), pages 461-469, XP002173578 ISSN: 0960-7412 cited in the application
- KILBY N J ET AL: "CONTROLLED INDUCTION OF GUS MARKED CLONAL SECTORS IN ARABIDOPSIS" JOURNAL OF EXPERIMENTAL BOTANY, OXFORD UNIVERSITY PRESS, GB, vol. 51, no. 346, May 2000 (2000-05), pages 853-863, XP001022119 ISSN: 0022-0957
- HOFF T ET AL: "A RECOMBINASE-MEDIATED TRANSCRIPTIONAL INDUCTION SYSTEM IN TRANSGENIC PLANTS" PLANT MOLECULAR BIOLOGY, NIJHOFF PUBLISHERS, DORDRECHT, NL, vol. 45, no. 1, 2001, pages 41-49, XP001022118 ISSN: 0167-4412
- HEERKLOTZ D ET AL: "THE BALANCE OF NUCLEAR IMPORT AND EXPORT DETERMINES THE INTRACELLULAR DISTRIBUTION AND FUNCTION OF TOMATO HEAT STRESS TRANSCRIPTION FACTOR HSFA2" MOLECULAR AND CELLULAR BIOLOGY, WASHINGTON, DC, US, vol. 21, no. 5, March 2001 (2001-03), pages 1759-1768, XP001022102 ISSN: 0270-7306

## Description

The invention relates to the field of transforming plant cells and the modification of plant genomes.

Current plant transformation technologies in general rely on antibiotic or herbicide resistance genes to provide a selective growth advantage to transformed cells in the presence of the selective agent, thus allowing the production of transgenic tissues. Transformation is in general seen as a process by which cells/tissues/plants acquire properties encoded on a nucleic acid molecule that has been introduced into cells using, but not limited to, microinjection, permeabilisation of the cell membrane, biolistics or *Agrobacterium tumefaciens* or *A. rhizogenes* infection.

Such newly introduced nucleic acid may not only comprise a coding region that encodes protein responsible for the desired acquired property per se, but may also comprise additional regulatory sequences such as a promoter, a nucleotide sequence usually upstream (5') of a coding region which controls the expression of that coding region by providing recognition for RNA polymerase and/or factors required for transcription to start at the correct site; a polyadenylation signal or terminator which is a nucleotide sequence usually located downstream (3') of a coding region which control addition of polyadenylic acid and termination of transcription or a regulatory nucleotide sequence controlling transcription initiation, elongation and termination.

The presence of markers or residues thereof in a transformed plant is in general considered undesirable. From another point of view elimination of a marker used would also be highly desirable, for the presence of a particular marker gene in a transgenic plant precludes the use of that same marker gene for subsequent modification of that plant. In theory, co-transforming a species with multiple desirable genes can solve this problem, a solution requiring accurate prediction of future markets. However, it is quite conceivable that breeders will want to be able to introduce additional traits into an already successful transgenic crop at a later stage. Unfortunately, access to alternative markers is restricted.

Excision of a (marker) gene using the site-specific recombination Cre/*lox* system from bacteriophage P1 was one of the first reported examples ofa marker removal system (Odell *et al.,* 1990; Dale and Ow 1991; Srivastava *et al.,* 1999). In this system, the marker and one of the flanking recombination sites are precisely and conservatively removed through the action ofCre recombinase when lox recombination sites with the same orientation flank it. One big disadvantage of this method is the additional effort needed to introduce the recombinase gene into the transgenic plants to get rid of a (marker) gene. This necessitates a second transformation with a construct containing the recombinase gene using selection with a second marker, or transgenic plants from the first round of transformation with the targeted marker have to be crossed with transgenic plants containing the recombinase gene. Even if that has been successful, the progeny from the transformation or the cross still has to be self-pollinated or crossed to an untransformed plant to segregate the two independent transgenic loci and to retrieve plants containing only the modified T-DNA with the desired transgene.

A second approach features transposition mediated re-positioning and subsequent elimination of marker genes from transgenic tomato (Goldsbrough *et al.,* 1993). In this system, the marker flanked by Ds elements is separated from the desired transgene by Ac transposase supplied in *cis.* If the genetic distance between the primary and secondary insertion sites created by the transposition is sufficient, recombination between them via selfing or outcrossing may yield marker-free progeny. Thus, both methods appear to be unsuitable for vegetatively propagated crops as the necessary sexual crosses would scramble the genome (Flavell *et al.,* 1992). However, in the second method segregation of the marker and the gene-of-interest is also possible without crossing as the *Ds* sometimes fails to re-integrate, but this occurs in only 10% of transposition events.

Ebinuma et al. (1996; 1997a) used the *ipt* gene in a bifunctional marker approach and were the first to show that the marker might be removed by a site-specific recombination system without having to rely on crossing, double transformations or abortive transposition events. During the first phase of their Multi-Auto-Transformation (MAT) procedure, transformed shoots emerging from the explants can be distinguished from untransformed shoots by their dwarf phenotype; a phenotype caused by excess cytokinin resulting from overexpression of the *ipt* gene residing on the T-DNA. The regeneration of untransformed cells is reduced by the lack ofcytokinin in the shoot regeneration medium, at least in those species requiring this hormone for regeneration. Following transfer to fresh medium, normal shoots emerge from these bushy shoots when they lose this marker through recombinase activity.

Remarkably, the R recombinase gene and the marker are both on the same T-DNA and production of the recombinase serves to remove both the marker as well as the recombinase, thus allowing repeated use of this system. Earlier reports utilising these systems in plants always supplied the recombinase separately and in *trans* in a second round of transformation or via a cross (see above). One other decisive difference between the approach taken by Ebinuma *et al.* (1996) and others lies in the use of the *ipt* gene as a marker for negative selection as well. The loss of the marker leads to normal growth that can be distinguished from the bushy material from which they emerge.

In the earliest MAT system featuring SR1 recombinase, its activity was expressed constitutively, but apparently it only rarely reached levels sufficient for removal ofthe target flanked by recombination sites. In a more recent version of the MAT system, the CaMV 35S promoter upstream of the R recombinase is replaced by the safener-inducible GST-II-27 promoter, which results in an increase in the frequency of *ipt*-shooty explants (Sugita *et al.,* 2000). However, paradoxically, the success of above methods is dependent on low recombinase activity.

Another approach towards the production of marker-free plants, which does not require crossing or selfing was described by Gleave *et al.* (1999). In this method plants were first transformed with a construct in which the DNA segment to be removed was flanked by *lox* recombination sites. Subsequently, two single-copy transformants were retransformed with agrobacteria containing a construct with Cre recombinase. Yet, the construct was not intended for stable integration and should transiently deliver sufficient recombinase activity to cause site-specific recombination. Therefore, the *hpt* marker on the binary plasmid was not used and hygromycin was omitted from the regeneration medium. As the *lox*-flanked DNA segment included the cytosine deaminase gene, removal could be easily scored by looking for fluorocytosine tolerance (FC; Stougaard, 1993). It turned out that a mere 0.25% of the shoots tested for recombinase-mediated deletion were FC-tolerant; the majority of them attributable to integrated Cre recombinase.

Not all systems for marker removal seem to require helper proteins like recombinase to induce deletion events. Zubko *et al.* (2000) reported about the production of marker-free transgenic tobacco via intrachromosomal recombination between bacteriophage lambda derived attP regions. However, the precision and frequency of the excision were low.

The invention provides a transformation system featuring the removal of the marker which is of utmost importance to plant biotechnology as a whole as it reduces the need for alternative markers, each with its own peculiarities and requiring its specific adaptations to the transformation protocol. The invention thus provides a method for obtaining essentially marker-free but transformed plant cells, plants or parts (such as roots, shoots, meristeme or callus material) thereof and progeny thereof and provides said essentially marker-free transformed plants or parts thereof.

Whereas in a preferred method provided the invention recombinase activity is tightly controlled and induction quickly leads to high activity to remove (all of) the inserted selectable marker and recombinase gene(s) at once, earlier attempts to induce recombinase activity cannot be considered to provide these advantages. For example, in WO0136595, recombinase activity is not regulated by a (let alone translationally fused) ligand binding domain (LBD) and no negative selection to obtain marker-free plant or plant cells is contemplated. Furthermore, in Lyznik et al (Plant Journal 8:177-186, 1995), disadvantagely a FLP recombinase, and what is more, a wild type form of the FLP gene instead of a plant-adapted version is used. Also, recombinase activity is not regulated by a (translationally fused) ligand binding domain, allowing essentially no negative selection to obtain marker-free plant (cells), and the recombinase gene is not removed along with marker gene.

In Sugita Koichi et al (Plant Journal 22:461-469,2000), recombinase activity is controlled by a chemically-induced promoter, and the wild type form of the R recombinase instead ofa plant-adapted version, making the method not suitable for removal ofantibiotic/herbicide resistance selectable markers, it relying on induction of phenotypic abnormalities due to the presence of the marker and return to normal phenotype in plants that have lost this particular marker. In other words, a kind of negative selection for the absence of the marker does exist.

In Zuo Jianri et al, Nature Biotech. 19:157-161, 2000, recombinase activity is not regulated by a translationally fused ligand binding domain. Instead the LBD is translationally fused to an artificial transcriptional activator. In this set-up, addition of an inductor activates the transcription factor, which then start transcription of the recombinase gene. The present invention provides regulation of the recombinase activity by an LBD that is direct, i.e. posttranslational, whereas indirect, i.e transcriptional regulation, as in the case of Zuo et al, essentially precludes negative selection to obtain marker-free plant (cells), for one it might be activated by phytoestrogens present in plant cells.

In EP0716147, recombinase activity is (low but) constitutively controlled by a 35S promoter, making the method not suitable for removal of antibiotic/herbicide resistance selectable markers, as it is relying on induction of phenotypic abnormalities due to the presence of the marker and return to normal phenotype in plants that have lost this particular marker, whereby a kind of negative selection for the absence of the marker does exist.

The importance of a system as provided herewith is even greater for 'recalcitrant'crops in view of the effort to achieve any transformation at all, let alone multiple transformations with different protocols for each step. The provision of a transformation system according to the invention allows repeated use of one and the same marker in sequential transformations and means that all can be performed using the same protocol (unless the previous transgene affects the transformation). In other words, once a fitting transformations system for a recalcitrant crop is functioning, lengthy trial-and-error research to optimise conditions with new, additional combinations of markers and selective agents will no longer be required to obtain new transformants.

In one embodiment, the invention provides the insight that inducible activation of a site-specific recombinase in plants allows inducible removal of the selectable marker following selection oftransgenic plants. In other words, the recombinase gene, (selectable) marker genes and any other genes or gene fragments whose presence is at some point in time no longer desired and that are according to the invention preferably located between recombination sites specific for said recombinase gene or fragment thereof are, upon inducing said recombinase acitivity, removed from the transformed plant cell, thereby leaving an essentially marker free transformed plant cell (and plants and progeny resulting thereof) as provided by the invention.

The relative success of the MAT system described by Ebinuma *et al.* (1996) depends on a relatively low recombinase activity, for high activity would greatly decrease transformation efficiency by premature of loss of marker gene activity. However, in a system utilising tightly regulated or inducible recombinase expression or localisation, as provided herewith, the high enzyme concentration desirable for achieving efficient recombination is permissible. The relatively low expression ofR recombinase in the MAT system may well be due to its relatively high A+U content leading to unfavourable codon usage and potential cryptic plant regulatory signals. For example, it was shown for the *Bacillus thuringiensis cryIA(b)* toxin - a gene with a high A+U bias - that complete modification of the coding sequence was required to achieve strong expression in transgenic tomato and tobacco plants (Perlak *et al.,* 1991). A synthetic gene with optimised codon usage and lacking ATTTA sequences and potential plant polyadenylation sites produced up to 100-fold higher protein levels than the wild type gene. Other examples of genes with high A+U bias and low expression in plant cells, like the genes encoding T4 lysozyme and *Klebsiella pneumoniae* cyclodextrin glycosyltransferase, demonstrate that low expression is not specific to *B. thuringiensis* genes [16, 39].

The invention provides a method for transforming a plant cell comprising providing said cell with a construct comprising a first nucleic acid encoding a first polypeptide with site-specific recombinase activity and a second nucleic acid encoding a polypeptide comprising a ligand binding domain (LBD) from a steroid receptor, who together encode a fusion polypeptide comprising site-specific recombinase activity and a functional ligand binding domain, and a third nucleic acid coding for a selectable marker, which can, after transformation, be excised by said recombinase. In general, each site-specific recombinase catalyses recombination between two identical enzyme-specific recombination sites, i.e. each recombinase has its own sites. In addition, several recombinases can also employ variant recombination sites besides the natural ones such as Cre/lox system of bacteriophage P 1, the FLP/FRT system of *Saccharomyces cerevisiae,* and the R/RS system of *Zygosaccharomyces rouxii.* LBD encoding sequences may be obtained from animal glucocorticoid, estrogen or androgen receptor genes. In addition, the invention provides a method further comprising providing said cell with a nucleic acid encoding a selectable marker flanked by said recombinase-specific recombination sites. Preferably, said recombinase-specific recombination sites flank nucleic acid encoding the recombinase gene or fragment thereof and/or the gene fragment encoding a polypeptide through which recombinase activity can be induced or enhanced as well, leaving an essentially marker-free transformed plant cell useful for regeneration into a transformed plant or its progeny containing only the desired trait. Selectable markers are for example provided in a non-exhaustive list of markers appended herewith or can be found in the art.

In a preferred embodiment of the invention, to maximise expression, the recombinase gene is resynthesised increasing its G+C content, for example from 41 to 49% and/or its frequency of favourable XXC/G codons from 41 to 63%, preferably without altering the amino acid sequence. For example, in the detailed description, the resulting synthetic gene has been fused to a ligand-binding domain or nuclear transport signal peptide.

The present invention as described above provides a method for producing inducible site-specific recombination of DNA in plant cells, and thus provides a transformed plant cell obtainable by said method. Said plant cell is for example first provided with a ligand-inducible site specific recombination system as described herewith. In general, a site-specific recombination system consists of two elements: a pair of DNA sequences (the recombination sites) and a specific enzyme (the site-specific recombinase). The site-specific recombinase catalyses a recombination reaction only between two particular recombination sites. Depending on the orientation of the recombination sites the sequences connecting the recombination sites will either be excised or inverted in the presence of the corresponding recombinase. The excised DNA segment is circularised and might be reintegrated in the presence of recombinase. However, if expression of the recombinase is restricted, the thermodynamically least favoured bimolecular integration reaction can be reduced.

The invention provides the insight that the activity ofrecombinase can be almost completely controlled by fusing it with the ligand-binding domain (LBD) ofa steroid receptor. Thus, recombinase is active only in the presence of specific ligands. This property allows the recombinase to be introduced in said plant cell together with DNA sequences encoding at least two recombination sites separated by a selected DNA segment, without being bothered by the premature excision or inversion of the selected DNA segment.

Cells can then be selected under the specific selective pressure indicated for the marker used. Subsequent excision is controlled by depriving said plant cell of its ligand-inducible recombinase activity comprising induction of said activity, and excising the DNA construct at its specific recombination sites, thereby excising the contained marker sequence and the nucleic acid encoding inducible recombinase activity. Preferably, said ligand comprises a steroid, in a preferred embodiment the invention provides a method for site-specific recombination in plant cells induced by specific steroids taken up by these cells.

The method for example provides transformation of plant cells with two DNA sequences containing recombination sites in such a way that they become inserted at different genomic locations. Alternatively, a selected DNA segment connects the two DNA sequences containing the recombination sites. In addition, a third DNA sequence comprising a gene encoding a site-specific recombinase translationally fused with a gene fragment encoding the ligand-binding domain (LBD) of a steroid receptor may also introduced into the plant cells by transformation. The recombinase needs to be specific, or at least to be selective for the recombination sites introduced as first and second DNA sequences. Several site-specific recombination systems have been described that function in plant cells, including but not restricted to the Cre/lox system of bacteriophage P1, the FLP/FRT system of *Saccharomyces cerevisiae,* and the R/RS system of *Zygosaccharomyces rouxii.* LBD encoding sequences may be obtained from animal glucocorticoid, estrogen or androgen receptor genes. The invention also provides a plant transformation vector comprising a construct having a first nucleic acid encoding a first polypeptide with site-specific recombinase activity and a second nucleic acid encoding a polypeptide comprising a ligand binding domain (LBD) from a steroid receptor which upon expression form a fusion polypeptide comprising site-specific recombinase activity and a functional ligand binding domain, and a third nucleic acid coding for a selectable marker, which can, after transformation, be excised by said recombinase.

In a preferred embodiment said first nucleic acid is at least partly derived from the R recombinase gene of *Zygosaccharomyces rouxii* (Araki *et al.,* 1992). To facilitate or maximise expression the invention preferebly provides a vector wherein said first nucleic acid has a G+C content of at least 45, more preferably of at least 48 percent.

Of course, when (removable) markers selection is desired, said vector according to the invention further comprises a nucleic acid encoding a selectable marker such as a monofunctional positive marker, a monofunctional negative marker and/or a bifunctional marker. A vector is preferred wherein said monofunctional negative or said bifunctional marker comprises cytosine deaminase activity, preferably wherein said bifunctional marker comprises neomycin phosphotransferase II and cytosine deaminase activity. In principle, negative selection for marker-free cells consists of the generation of toxic proteins/peptides controlled by inducible promoters. In fact, however, the obligatory use of an inducible protein to control proteins that are toxic as such effectively ruins the possibility of using the negative marker in a fusion with the positive (antibiotic resistance) marker. The latter would not be active until induction, i.e. selection oftransgenic cells would be impossible. The cytosine deaminase (codA) gene used in the monofunctional negative or bifunctional marker as provided by the invention herein allows the use of constitutive promoters, because the encoded protein is only toxic to cells in the presence of a non-toxic substrate that is converted to a toxin by the action of the enzyme.

It is preferred that the DNA segment to be removed does include the hybrid recombinase-LBD gene. In one embodiment, the vector comprising a nucleic acid sequence encoding for example a hybrid recombinase-LBD gene connects two recombination sites with the same orientation and further comprises a promoter that drives expression of the hybrid gene in plant cells, and a polyadenylation signal. Both the promoter controlling the expression of the hybrid gene as well as the terminator may reside outside the region flanked by recombination sites. In this case, site-specific recombination causes deletion of the third DNA sequence linking the two recombination sites. If the same DNA sequence connects two recombination sites with opposite orientation, site-specific recombination leads to inversion of the DNA sequence encoding the hybrid gene driven by a promoter active in plant cells and a polyadenylation signal.

In another embodiment, the DNA sequence connecting the two recombination sites with the same orientation does not only encode the hybrid gene and its promoter and terminator as described above, but it also comprises a second gene encoding a marker or trait that has to be removed at a later stage. Neither the promoters nor the term inators need to be part of the sequence connecting the recombination sites. Expression of the second gene is under the control ofa plant promoter and transcription is terminated by a polyadenylation signal. Site-specific recombination causes excision of both genes and other sequences present between the two recombination sites.

More preferably, the DNA introduced into plant cells does not merely contain a hybrid recombinase-LBD gene, a marker gene and their respective regulatory sequences flanked by recombination sites with the same orientation, but also one or more genes encoding agriculturally or horticulturally important traits together with their regulatory sequences located outside the region flanked by recombination sites. Typical marker genes confer resistance to hygromycin, kanamycin, bleomycin, sulfonylurea, and phosphinothricin. Transformed cells are obtained by selecting for resistance towards a selective agent depending on the marker that was used. The transformed cells can be regenerated into organs or whole plants before being subjected to a treatment aimed at recombinase activation. Alternatively, undifferentiated transformed cells are contacted with a ligand specific for the LBD that was used in the transformation vector.

In a preferred embodiment, the LBD is derived from the rat glucocorticoid receptor the starting material for the recombinase activation treatment is a transgenic plant comprising a desired trait. Dexamethasone can be applied in several ways.

### Figure legends

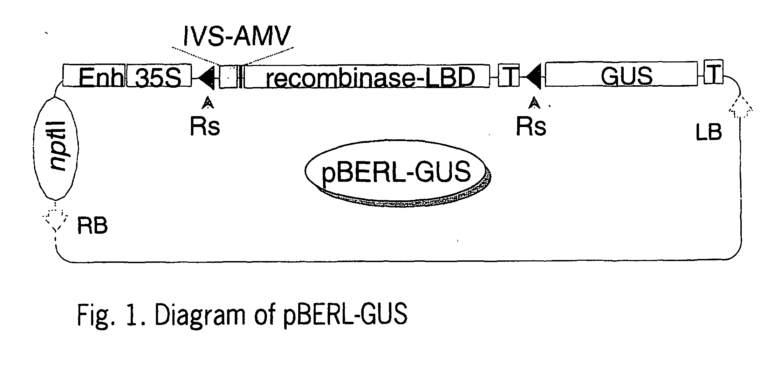

Fig. 1. Diagram of pBERL-GUS. Enh = CaMV 35S enhancer; 35S = CaMV 35S promoter; Rs = recombination site; IVS-AMV = alfalfa mosaic virus translational enhancer preceded by intron 5 of the potato granule-bound starch synthase gene; recombinase = synthetic R recombinase; LBD = rat glucocorticoid receptor ligand binding domain; T = nos terminator; *npt* II = neomycin phosphotransferase II; GUS = β-glucuronidase; LB and RB = left and right border

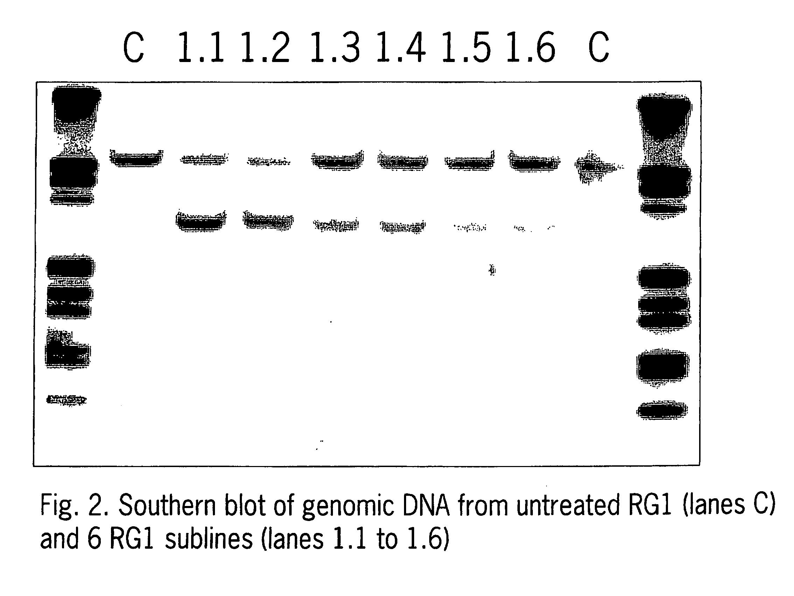

Fig. 2. Southern blot of *Eco* RI-digested genomic DNA from untreated (lanes C) and DEX-treated (lanes 1.1 to 1.6) pBERL-GUS transformed potato lines probed with DIG-labeled GUS-fragment.

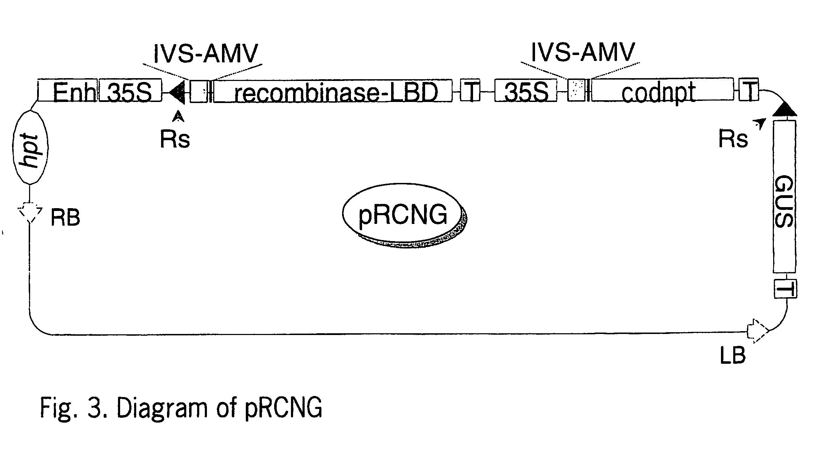

Fig. 3. Diagram of RCNG-construct. Enh = CaMV 35S enhancer; 35S = CaMV 35S promoter; Rs = recombination site; IVS-AMV = alfalfa mosaic virus translational enhancer preceded by potato *gbss* intron 5; recombinase = synthetic R recombinase; LBD = rat glucocorticoid receptor ligand binding domain; T = nos terminator; *cod A* = cytosine deaminase; *npt* II = neomycin phosphotransferase II; GUS = ß-glucuronidase; *hpt* = hygromycin phosphotransferase; LB and RB = left and right border.

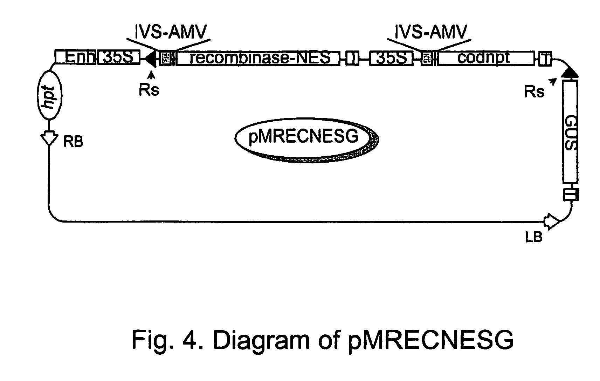

Fig 4. Diagram of pMRECNESG construct. Enh = CaMV 35S enhancer; 35S = CaMV 35S promoter; Rs = recombination site; IVS-AMV = alfalfa mosaic virus translational enhancer preceded by potato *gbss* intron 5; recombinase = synthetic R recombinase; NES = nuclear esport signal peptide; T = nos terminator; *cod A* = cytosine deaminase; *npt* II = neomycin phosphotransferase II; GUS = β-glucuronidase; *hpt* = hygromycin phosphotransferase; LB and RB = left and right border

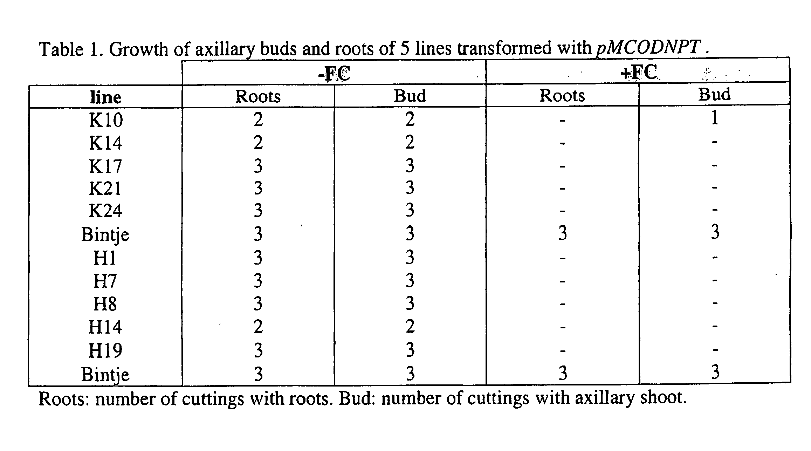

Fig. 5 = Table 1

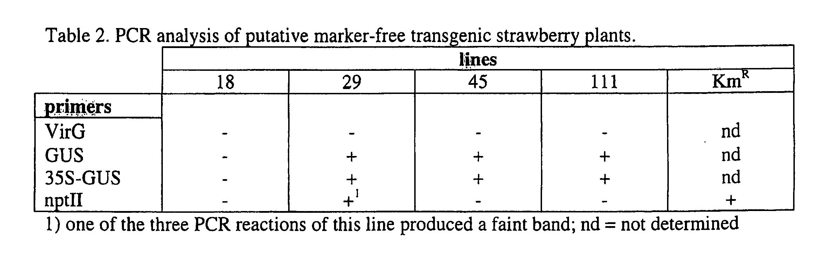

Fig. 6 = Table 2

### Detailed description

### Constructs

*pBIN35Snos.* The plant transformation vector for insertion of the recombinase and GUS genes was derived from pBIN19 by introducing a double CaMV 35S promoter and a nopaline synthase gene *(nos)* terminator (Bevan, 1984). Two *Sac*I*-Eco*RI fragments containing the *nos* terminator from pBI221 were cloned into *Sac*I digested pMTL23 (Chambers *et al.,* 1988). *Bam*HI*-Eco*RI double digestion of this clone produced a fragment containing one *nos* terminator that was ligated to similarly digested pBIN19 giving rise to pBIN19nos. A CaMV 35S promoter with a duplicated enhancer region was obtained as follows: the CaMV 35S promoter from pRok1 was cloned into pUC19 yielding pPCaMV (Baulcombe *et al.,* 1986; Yanisch-Perron *et al.,* 1985). The enhancer fragment from the 35S promoter was obtained by subcloning a *Hind*III*-Eco*RV fragment from pBI121 into pBluescript SK+ (Stratagene Cloning Systems, La Jolla, CA) giving pCaEH (Kay *et al.,* 1987). The enhancer containing fragment and the complete promoter were combined into an enhanced promoter by ligating the corresponding fragments from *Hind*III*-Eco*RI digested pCaEH and *Eco*RI*-Xba*I digested pPCaMV into *Hind*III*-Xba*I digested pBI121, The enhanced 35S promoter was isolated from this construct by *Hind*III*-Bam*HI double digestion and cloned into pBIN 19nos giving rise to pBIN35Snos.

*pAMV-1.* pAMV-1 is a pMTL23 derivative containing the translation enhancing 5' untranslated region (UTR) of the alfalfa mosaic virus (AMV) cloned into its *Bg*/*II-* and *Nco*I-sites (Jobling and Gehrke, 1987), pAMV-1 was obtained by ligating duplexes consisting of T4 polynucleotide kinase phosphorylated oligonucleotides 5'-GATCTGTTTTTATTTTT-AATTTTCTTTCAAATACTTCCAC-3' and 5'-CATGGTGGAAGTATTTGAAAGAAAAT-TAAAAATAAAAACA-3' into *Bgl*II*-Nco*I digested pMTL23. The resulting vector contains one mutation, i.e. the underlined C-residue has been replaced by an A-residue.

*pIVSAMV.* Intron 5 from the potato granule-bound starch synthase gene was isolated from *Solanum tuberosum* cv. Bintje by PCR using the following oligonucleotides: 5'-CTGGAAGATCTGGACAATCAACTTAG-3' and 5'-GCTACGGATCCAATTCAAAACT-TTAGG-3'(Van der Leij *et al.,* 1991). The purified fragment was cut with *BamHI* and *Bgl*II and cloned into *Bgl*II digested pAMV-1 (Rouwendal *et al.,* 1997). The sequence of the cloned intron was verified by dideoxy sequencing using the ALF system (Pharmacia Biotech).

*pREC.* The strategy for constructing the synthetic recombinase gene was based on the overlap extension PCR method using 24 long oligonucleotides as the starting material (Ho *et al.,* 1989). The first step consisted of 12 amplification reactions with pair-wise combinations of overlapping sense (odd numbered: 1-23) and (even numbered; 2-24) antisense oligos each with an average size of 81 nt (Rouwendal *et al.,* 1997). The reactions were carried out as follows: 94 °C for 30 s, 45 °C for 15 s, 72 °C for 15 s for 10 cycles in standard *Pfu* DNA polymerase buffer with 20 pmol of each oligo and 0.5 u *Pfu* in a 25 microliter final volume. The reaction products were purified by Qiaquick extraction (QIAGEN GmbH) and redissolved in 10 mM Tris-HCl pH 8.0, 0.1 mM EDTA (T₁₀E_{0.1}). In the second step, approximately 20% of the double stranded reaction products were again coupled by PCR overlap extension under the following conditions: 94 °C for 30 s, 35°C for 1 m in, 72 °C for 45 s for 25 cycles in Advantage KlenTaq DNA polymerase buffer (Clontech) with 10 pmol of oligos 1 plus 4 (segment A; positions 1-275), 5 plus 8 (segment B; positions 256-527), 9 plus 12 (segment C; positions 508-779), 13 plus 16 (segment D; positions 760-1031), 17 plus 20 (segment E; positions 1012-1283) and 21 plus 24 (segment F; positions 1264-1489) and 0.5 µL Advantage KlenTaq in 25 µL final volume. The products were again purified by Qiaquick extraction and redissolved in T₁₀E_{0.1}. Subsequently, cloning of the PCR products was done by employing the additional 3' adenosine to insert them into a pGEM5Zf-derivative with 3'T-overhangs created by *Xcm*I digestion (Schutte *et al.,* 1997). Several independent clones were sequenced for each of the 6 segments to identify one without errors. This was successful for all segments except segment B. The two errors in segment B were repaired using a combination of overlap extension and megaprimer mutagenesis methods (Sarkar and Sommer, 1990). Clones of the remaining 5 segments were subjected to PCR with short terminus specific oligos and all 6 PCR fragments were then fused in a series of overlap extension reactions using *Pwo* DNA polymerase. The resulting full-length product was digested with *Nco*I and *Bam*HI and cloned into pAMV-1. Sequencing of 3 different clones revealed 1 clone with only 1 error. The error was corrected by overlap extension mutagenesis and the product digested with *NcoI* and *BamHI* and cloned into *pIVSAMV.* Again, dideoxy sequencing was used to check for errors.

*pLBD.* The LBD from the rat GR (amino acid residues 512-795) was obtained from clone 6RGR by overlap extension mutagenesis of 2 PCR fragments generated with primers 5'-CTCTG-AGATCTACAAAGAAAAAAATCAAAGGGATTCAGC-3' and 5'-CTGGGAAC-TCAATACTCATG-3'and with primers CTTAGGGATCCAGTCATTTTTGATGAAACA-GGAG-C-3' and 5'-CATGAGTATTGAGTTCCCAG-3', respectively (Miesfeld *et al.,* 1986). The mutation removed an *Eco*RI site. The product was cloned into pMTL23 and its sequence was verified by dideoxy sequencing.

*pCODNPT.* The *Escherichia coli* cytosine deaminase gene *(cod*A*)* was isolated from strain JM109 by PCR using primers 5'-GTGAACCATGGCTAATAACGCTTTACAAACAA-3' and 5'-GCAGTGGATCCACGTTTGTAATCGATGG-3'. Following digestion with *Nco*I and *Bam*HI the PCR product was cloned into the *Nco*I and *Bam*HI sites of pIVSAMV. The *npt*II gene was isolated by PCR from pBIN19 using primers 5'-TCGCAGATCTGAACAAGA-TGGATTGCACG-3'and 5'-GCTCAGGATCCCGCTCAGAAGAACTCGTC-3'. This PCR fragment was digested with *Bgl*II and *Bam*HI before being cloned into the *Bgl*II and *Bam*HI sites of pMTL23. The sequences of the two clones were verified by sequencing before continuing the construction of a hybrid gene. The fusion gene was made by cloning the *Bgl*II*-Bam*HI fragment comprising the *gbss* intron, the AMV enhancer and the *cod*A gene into the *Bgl*II site upstream of the *npt*II gene.

*pMCODNPT.* The *Bgl*II*-Bam*HI fragment consisting of the hybrid *codA-npt*II gene with its expression-enhancing 5'UTR was inserted into the *Bam*HI site of pMOG-EGUSn. The final construct pMCODNPT was introduced into *A*. *tumefaciens* LBA4404 by electroporation (Nagel *et al.,* 1990).

*p35S.* The CaMV 35S promoter was isolated from pBIN35Snos by PCR using primers 5'- GCGACAGATCTAATGCTAACCCACAGATGG-3' and 5'-GCGACGGATCCCCTCTC-CAAATGAAATGAAC-3'. Following digestion with *Bgl*II and *Bam*HI the fragment was cloned into *Bgl*II*-Bam*HI digested pMTL23. Sequencing was used to check for PCR errors.

*pNOSt.* The nos terminator was obtained by PCR with primers 5'-GTGACAGATCTCGAATTTCCCCGATCGT-3' and 5'-CCAGTGGATCCCCGATCT-AGTAACATAG-3' using pBIN35Snos as template. The *Bgl*II*-Bam*HI digested fragment was cloned into *Bg*/II*-Bam*HI digested pMTL23.

*pRsRECLBDnosRs.* The LBD from the rat GR was translationally fused to the C-terminus of the synthetic R recombinase gene by inserting it as a *Bgl*II*-Bam*HI fragment into *Bam*HI digested pREC. Clones containing the LBD in the correct orientation were found by PCR and designated pRECLBD. The fusion created 2 additional aa residues between the C-terminus of the recombinase and the N-terminus of the LBD. The nos terminator was obtained by PCR with primers 5'-GTGACAGATCTCGAATTTCCCCGATCGT-3' and 5'-CCAGTGGAT-CCCCGATCTAGTAACATAG-3'using pBIN35Snos as template. The resulting fragment was digested with *Bgl*II and *Bam*HI and cloned into the *Bam*HI site of pRECLBD. Clones with the nos terminator in the right orientation were identified by PCR and designated pRECLBDnos. The Rs was also isolated by PCR using primers 5'-AGGCGAGATCTTATCACTGT-3'and 5'-GTCACGGATCCACGATTTGATGAAAG-AAT-3'. This short PCR product was cut with *Bgl*II and *Bam*HI and cloned into the *Bam*HI site ofpRECLBDnos. Constructions with Rs in the right orientation were selected using PCR. The *Bgl*II*-Bam*HI digested Rs PCR product was also used to insert an Rs sequence into the *Bgl*II site upstream of the recombinase-LBD hybrid gene. Dideoxy sequencing was used to verify the sequences of the nos term inator and the Rs sequences and to check whether the Rs sequences were directly repeated.

*pBERL-GUS.* The recombinase-LBD hybrid gene including its flanking Rs sequences was isolated from pRsRECLBDnosRs by *Bgl*II*-Bam*HI digestion and cloned into the *Bam*HI site upstream of the GUS in pCeGN. pCeGN is a pBI221-derivative in which the CaMV 35S promoter has been replaced by its enhanced version from pBIN35Snos (Jefferson, 1987). Constructions with the insertion in the right orientation were selected by PCR. Next this clone was treated with *Eco*RI, which cut one restriction site between the enhancer and the CaMV 35S promoter upstream of the recombinase-LBD hybrid gene and a second site downstream of this combination at the end of the original pUC19 multiple cloning site. The fragment comprising the promoter, the hybrid gene and GUS was isolated and inserted into *Eco*RI digested pBIN35Snos from which the resulting promoter and nos terminator containing fragment had been removed following the digestion. Correct insertion of the large fragment was verified by PCR and restriction digestion. The final construct pBERL-GUS was introduced into *Agrobacterium tumefaciens* LBA4404 by electroporation (Nagel *et al.,* 1990).

*pMOG-EGUSn.* The pBIN35Snos vector was digested partially with *Eco*RI and completely with *Hind*III. The resulting fragment comprising the enhanced 35S promoter and the nos term inator was cloned into *Hind*III-*Eco*RI digested pMOG22, a vector similar to pBIN19 with a CaMV 35S driven hygromycin phosphotransferase *(hpt)* gene as plant selectable marker instead of the *nptII* gene (kindly provided by MOGEN international b.v.).

*pTOPORs.* The recombination site Rs was isolated from *Zygosaccharomyces rouxii* by PCR using primers 5'-AGGCGAGATCTTATCACTGT-3'and 5'-GTCACGGATCCACGAT-TTGATGAAAG-AAT-3'. This short PCR product was directly cloned into pCR2.1 using a TA cloning kit (Invitrogen) and the resulting clone was sequenced.

*pRCNG.* The LBD from the rat GR was translationally fused to the C-term inus of the synthetic R recombinase gene by isolating it as a *Bgl*II*-Bam*HI fragment from pLBD and inserting it into *Bam*HI digested pREC. Clones containing the LBD in the correct orientation were found by PCR and designated pRECLBD. The fusion created 2 additional aa residues between the C-terminus of the recombinase and the N-terminus of the LBD. Next, a nos terminator containing fragment from pNOSt was obtained by digestion with *Bgl*II and *Bam*HI and cloned into the *Bam*HI site downstream of the LBD in pRECLBD. Clones with the nos terminator in the right orientation were identified by PCR and designated pRECLBDnos. The CaMV 35S promoter required for controlling the hybrid CODNPT marker gene was cut from p35S using *Bgl*II and *Bam*HI and cloned into the *Bam*HI site downstream of the nos terminator in pRECLBDnos. Again, PCR was used to identify clones with the insertion in the desired orientation. Likewise, the *Bgl*II*-Bam*HI fragment containing the CODNPT gene from pCODNPT and including the 5' UTR consisting of the AMV translational enhancer and a plant intron sequence was inserted into the *Bam*HI site downstream of the CaMV 35S promoter. Once more, the *BglII-Bam*HI digested nos terminator fragment from pNOSt was used, now to insert it into the *Bam*HI site downstream of the marker gene. Two recombination sites were inserted as *Bgl*II*-Bam*HI fragments isolated from pTOPORs, one into the *Bgl*II site upstream of the recombinase-LBD gene and another into the *Bam*HI site downstream of the nos terminator. Sequencing was used in both cases to check the orientation of the two sites. The pRCNG vector was made by inserting the large *Bgl*II*-Bam*HI fragment containing the recombinase and the marker genes flanked by Rs sequences into the *Bam*HI site of pMOG-EGUSn.

### Plant transformation and tissue culture

*Potato transformation.* Potato shoots (*Solanum tuberosum* cv. Saturna or Bintje) required for transformation experiments and the resulting transgenic plants were maintained in tissue culture at 23°C under a 16h light/ 8 h dark regime on MS medium (Murashige and Skoog, 1962) containing vitamins, 0.8 % (w/v) agar, and 3% (w/v) sucrose. Transgenics were grown in the same medium supplemented with kanamycin (100 mg/L) and cefatoxim (200 mg/L) (Murashige and Skoog, 1962). Stem explants of axenically growing potato plants (*Solanum tuberosum* cv. Bintje) were transformed with *A. tumefaciens* strain LBA4404 harbouring the binary vectors essentially as described by Edwards *et al.,* (1991), except for the use of stem segments instead of leaf disks and for the use of cefatoxim (200 mg L⁻¹) instead ofaugmentin.

*Strawberry transformation.* Strawberry plants (*Fragaria x ananassa* cv Calypso) required for transformation experiments were grown in tissue culture for 4 to 6 weeks on propagation medium containing MS medium including vitamins (2.2 g/L), 3% (w/v) sucrose, 0.9 % (w/v) agar, supplemented with BAP and IBA both at concentrations of 0.1 mg/L (pH 5.8) in a growth chamber at 25°C under a 16 h light/ 8 h dark regime. For transformation leaf discs 7 mm in diameter were cut then and transferred to plates with cocultivation medium (2.2 g/L MS medium including vitamins, 3% glucose, 0.4% phytagel, 0.2 mg/L NAA, 1.0 mg/L TDZ and 100 µM acetosyringone, pH 5.8). A suspension of strain *A. tumefaciens* AGL0 harbouring the binary plasmid was obtained by overnight culturing in 10 mL LB medium supplemented with kanamycin (50 mg/L) and rifampicin (50 mg/L) in a 50 mL tube in a rotary shaker at 28°C. The bacteria were pelleted by centrifugation at 2500 g and after decanting the supernatant the pellet was resuspended in 40 mL MS medium (2.2 g/L) containing 3% glucose and 100 µM acetosyringone (pH 5.2). For infection, 20 mL ofagrobacterial suspension was poured in a Petri dish containing leaf discs lying on cocultivation medium. After 10-20 minutes the leaf discs were blotted dry on a sheet of filter paper and placed on top ofa disc of Whatman filter paper (grade 1, Ø 8.5 cm) covering fresh cocultivation medium in a Petri dish. After cocultivation for 4 days at 21°C in the dark, the leafdiscs were transferred to regeneration medium, i.e. cocultivation medium supplemented with cefotaxim (250 mg/L) and kanamycin (100 mg/L) and grown at 25°C in a 16 h light/8 h dark regime. The leaf discs were placed on fresh plates every 4 weeks and regenerated shoots were put in jars containing MS-medium (4.4 g/L) with 3% sucrose and 0.9% agar and kanamycin (50 mg/L) and cefotaxim (250 mg/L) for further propagation. Shoots that grew well and produced roots on this medium were again transferred to the same medium but now without kanamycin and cefotaxim

### Genomic DNA isolation and analysis

Potato genomic DNA was isolated from 1 g samples of tissue-culture grown plants using the Nucleon Phytopure plant DNA extraction kit with an additional centrifugation step for 45 min at 30,000g in a Sorvall SS34 rotor to clarify the supernatant of the first centrifugation. *Eco*RI-digested DNA was separated in a 0.8 % agarose gel and the gel was blotted for Southern analysis as described previously (Allefs *et al.,* 1990). The probe was labeled by PCR with specific primers in the presence of DIG (digoxigenin) DNA labeling mix consisting of 100 µM of dATP, dGTP, dCTP (each) and 65 µM dTTP, 35 µM DIG-11-dUTP (Roche Molecular Biochemicals). Hybridisation with 10 ng/mL DIG-labeled probe was carried out overnight at 42°C in Ultrahyb buffer as described by the manufacturer (Ambion). The next day the blot was first washed twice for 5 min at room temperature in 2xSSC, 0.1% SDS and then twice stringently for 15 min at 65°C in 0.5xSSC, 0.1% SDS. The blot was then prepared for anti-DIG-AP mediated detection of hybrids using the DIG wash and block set as described. The actual visualisation of alkaline phosphatase-conjugated antibodies bound to the DIG-labeled hybrids was done using CDP-Star (Roche Molecular Biochemicals) and a Berthold NightOWL for detection of the luminescence.

Strawberry genom ic DNA for PCR analysis was isolated from leaves of tissue cultured plants according to Haymes (1996). Primer pairs used in the analyses were as follows: a) detection of *npt*II gene: 5'-TGGGCACAACAGACAATCGGCTGC-3' and 5'-TGCGAATCGGGAGCGGCGATACCG-3'; b) detection of GUS: 5'-CTGTAGAAACCCC-AACCCGTG-3'and 5'-CATTACGCTGCGATGGATCCC-3'; c) detection of *vir*G: 5'-GCC-GGGGCGAGACCATAGG-3' and 5'-CGCACGCGCAAGGCAACC-3'; d) detection of recombination event: 5'-CCACTATCCTTCGCAGACC-3' and 5'- TATCTGCATCGGCG-AACTGA-3'.

### RESULTS

### Design and synthesis of a modified R recombinase gene

Efficient operation of a conditional recombinase-mediated DNA removal system requires relatively high enzyme levels. Previous work with the Z. *rouxii* wild type R recombinase gene suggested that it was not highly expressed in plants (Onouchi *et al.,* 1991, 1995). As this might be due to the high A+U bias present in the gene, we decided to increase the G+C content from 41% to 49%, i.e. a value much closer to those observed in highly expressed plant genes from dicotyls like those encoding the small subunit of RuDP carboxylase (Rouwendal *et al.,* 1997). The strategy for synthesis of the recombinase gene was quite similar to the one used previously for modifying codon usage of the gene encoding green fluorescent protein (Rouwendal *et al.,* 1997). It involved the coupling of long overlapping oligos in the first step followed by overlap extension amplification in the second step. Contrary to the previous report, 6 intermediary products of about 250 bp comprising 4 long oligos each were now cloned first and then sequenced, before continuing with the synthesis procedure. The sequencing was used to check for errors, which were repaired if necessary. Selected clones were used as templates to obtain the six PCR fragments with correct sequences, which together spanned the whole gene. These amplifications were carried out with short oligos instead of the long ones that were used for the original reactions, because it appeared that most errors were due to the long oligos used as starting material and not to the PCR as such. Next the PCR fragments were utilised to synthesise a full-length recombinase gene in a series of overlap extension reactions. The products were cloned and several were sequenced. The single error in one of these clones was repaired using overlap extension mutagenesis again, and the *Bam*HI*-Bgl*II digested recombinase fragment was cloned into vector *pIVSAMV* placing intron 5 of a potato *gbss* gene and the AMV translational enhancer in its 5' UTR. The aa sequence from the resulting recombinase deviated from the wild type R gene near the start to accommodate inclusion of an *Nco*I site, and it did not have a stop codon to allow a translational fusion with the rat GR LBD.

### Construction ofa plant transformation vector for testing steroid-inducible recombinase activity

LBDs of different members from the steroid receptor family can mediate conditional recombinase activation in transfected mammalian cell lines, when they are C-terminally fused to this gene. Steroid-mediated activation of transcription factors in transformed plant cells has usually been accomplished by the LBD from the rat GR (Lloyd *et al.,* 1994; Aoyama and Chua, 1997). Lloyd *et al.* (1994) also tested the LBD of the estrogen receptor, but it provided poor control of the activity of the transcription factor to which it was fused. Perhaps, the presence ofphytoestrogens caused constitutive induction (Kurzer and Xu, 1997). Nevertheless, the estrogen binding domain was used quite successfully by Zuo *et al.* (2000) to control the activity of a transactivator in transgenic plants.

In order to test the applicability of a steroid receptor domain mediated activation of recombinase activity in plants, we began by constructing the RG-construct in which recombinase-catalysed removal of the gene encoding this enzyme would lead to GUS expression. Starting from pREC, the LBD, nos terminator and Rs sequence were added sequentially as as *Bgl*II*-Bam*HI fragments into the *Bam*HI site of pREC and its successors. Following insertion of the second Rs sequence into the *Bgl*II site upstream of the recombinase gene, the entire segment flanked by Rs was inserted into a pBIN 19-derived vector between the enhanced CaMV 35S promoter and the GUS gene creating pBERL-GUS (Fig. 1).

### Testing pBERL-GUS

The performance ofthe recombinase-LBD gene in a plant cell was tested using transformants obtained from transformation of potato cv. Bintje with A. *tumefaciens* LBA4404 carrying pBERL-GUS. Five lines were randomly selected from regenerated shoots that had rooted. GUS-staining of untreated leaf and stem tissues showed that lines RG2 and RG5 were strongly and uniform GUS-positive, lines RG1 and RG3 were more or less GUS-positive with 10-15% ofthe cells scattered throughout the leaf tissue staining blue and the last one - RG4 - was very faintly GUS-positive. The presence of strong GUS-staining lines is unlikely to be due to premature excision of the recombinase in the bacterial host, because we were unable to detect such events in 20 colonies picked at random (data not shown). Thus, it would seem that the premature excisions causing the GUS staining may occur at an early stage during the regeneration of a transgenic shoot. The blue spots in GUS-stained leaves of lines RG1 and RG3 indicate that premature excisions also occur to some extent in these lines. A Southern blot of untreated RG1 plants revealed a very faint band corresponding to a fragment created by recombination (data not shown). The same Southern also showed that the weakness of the GUS-signal in RG4 might be due to the multicopy character of the insertions.

Treating leaves from lines RG1 and RG3 with DEX for 24 hours induced a strong and quite uniform GUS stain indicating that the recombinase is expressed and DEX-inducible. Furthermore, it shows that recombinase can also function in mitotically inactive cells.

Single-node cuttings from RG1 potato shoots were incubated overnight in liquid MS30 with 10 µM DEX and then transferred to solid MS30. The axillary buds were allowed to grow into shoots and each of these shoots was propagated separately and designated as a subline. GUS staining performed on leaves from the sublines showed that almost all plants were chimaeric indicating that recombination had been incomplete. Genomic DNA was isolated from each ofthe sublines and untreated RG1 to characterise the nature and extent ofthe genomic alterations caused by recombination. Fig. 2 shows clearly that GUS staining is accompanied by the expected deletion of a 2.9 kbp fragment. The blot also shows that the extent of recombination, as reflected by the amount of 3.0 kbp *Eco*RI fragment, varies considerably between sublines.

### Assessment of a dual selectable marker consisting of the E. coli codA and the Tn5 nptII genes

The construction ofthe hybrid *codA-npt*II gene caused a few alterations in the coding sequences ofthe two participants. In the *codA* gene the serine at position 2 changed into alanine and in the *npt*II gene the N-terminal methionine and isoleucine were mutated to glycine and serine, respectively.

Potato stem explants ofcv Bintje were transformed with pMCODNPT and transformants were selected using kanamycin or hygromycin as the selective agents. The transformation yielded many transgenic shoots; both on kanamycin and on hygromycin 25% ofthe explants developed one or more shoots. This strongly suggested that the NPTII encoded by the C-terminal part ofthe hybrid gene was produced and active.

The kanamycin resistance ofthe shoots produced in the transformation rendered it very likely that N-terminal part ofthe hybrid protein was also being produced. In order to test its activity three single-node cuttings from five randomly selected independent lines originating from both selective media were transferred to media with and without 5-FC.

The results in Table 1 provide clear evidence that all transgenic lines are sensitive to FC, which demonstrates that cytosine deaminase activity is present in all ten transgenic lines and not in the untransformed cv. Bintje control cuttings. In other words, both components ofthe hybrid marker gene are active.

### Construction ofa plant transformation vector for selectable marker removal using steroid-inducible recombinase activity

As it was clear now that the recombinase-LBD hybrid gene functioned in plant cells, its suitability in a system for elimination of plant selectable markers subsequent to the transformation was assessed. In addition, a potentially suitable dual selectable marker had been identified as well. Analogous to the construction of pBERL-GUS, many of the elements of the new plant transformation vector required for testing marker removal, i.e. the LBD, nos terminator, CaMV 35S promoter, CODNPT, nos terminator and one Rs sequence - were again assembled sequentially by cloning each element as a *Bgl*II*-Bam*HI fragment into a *Bam*HI site starting with pREC and continuing with the *Bam*HI site of each of its successors. Then, after insertion of a second Rs sequence into the single *Bgl*II site upstream of the chimaeric recombinase-LBD gene, the large *Bgl*II*-Bam*HI insertion comprising the recombinase-LBD gene together with the CaMV 35S controlled marker gene, was positioned between the enhanced CaMV 35S promoter and the GUS gene in a pMOG22-derived vector (Fig. 3).

Hence, a promoter that should lead to high recombinase-LBD levels drove the hybrid gene. Induction ofrecombinase activity should cause the removal of the Rs flanked DNA segment including one halfofeach Rs. As a result, GUS would be placed under the immediate control of the enhanced CaMV 35S promoter.

### Testing pRCNG

Strawberry cv. Calypso was used as a host for *A. tumefaciens-mediated* transformation of construct pRCNG. Four weeks after the agrobacterial infection, 125 leaf discs displaying the onset of shoot formation were incubated overnight in liquid medium with 10 µM dexamethasone (DEX) before being transferred to fresh regeneration plates supplemented with 10 µM DEX and 150 µg/mL fluorocytosine (FC). In the course of the next three to four weeks, most of the leaf discs failed to yield any shoots and only four putative marker-free transgenic shoots were obtained, despite the fact that many more shoot initials appeared to be present at the start of the incubation. The loss of the marker and the transgenic nature of these lines and a kanamycin resistant (Km^{R}) control line was examined by PCR analysis using primers for the detection of *vir*G, *nptII,* GUS and the stable recombination product. PCR with the 35S-GUS primers would only yield a (small) product if the DNA segment flanked by Rs sequences had been removed.

The PCR analysis revealed that two, possibly three, of the lines surviving the FC selection were transgenic, had lost the marker and contained the expected DNA sequence with a GUS gene under the control of the CaMV 35S promoter. The lack of signal with the *vir*G primers indicates that the positive signals were not due to agrobacteria that escaped the cefatoxim regime. Therefore, we conclude that marker-free transgenic plants have been obtained.

At first sight the fact, that plant cells are able to accommodate the regulatory mechanism of a mammalian glucocorticoid receptor may seem to be quite remarkable. The glucocorticoid-dependent activity of an adenovirus E1A product was the first example ofa heterologous regulatory factor controlled by the LBD ofa member of the steroid receptor family (Picard *et al.,* 1988). Several years later, this regulatory mechanism was utilised in plants to control activity of the maize transcription factor R in *Arabidopsis* by fusing it with the LBD from the rat glucocorticoid receptor (Lloyd *et al.,* 1994). In addition, a novel system for glucocorticoid-inducible plant transcription was developed based on a chimaeric transcription factor comprising the DNA-binding domain of the yeast transcription factor GAL4, the transactivating domain of the herpes viral protein VP16 and the LBD of the rat glucocorticoid receptor (Aoyama *et al.,* 1995; Aoyama and Chua, 1997). Steroid receptors are direct signal transduction systems in which binding of the hormone signal to the LBD creates a hormone-activated form that alters transcription rates of specific genes (Yamamoto, 1985). Separate domains of the receptors are responsible for signal reception and subsequent DNA binding. The LBD represses the transcriptional activity of the receptor in the absence of hormone, it binds hormone and it determines hormone-dependent activation of transcription (for review: Pratt, 1993). It is now clear that the steroid receptors exist in multiprotein cytosolic complexes consisting of hsp90, hsp70, p60 and other proteins, several ofwhich are sufficiently conserved to explain the assembly of GR in these functional multiprotein complexes in plants (Stancato *et al.,* 1996). Recent observations on the nature of the multicomponent protein complexes suggest that they may act as chaperone machinery consisting of selfassembling protein-folding structures called foldosomes (Hutchison *et al.,* 1994). Given the abundance of the proteins comprising the hsp90 chaperone system and the apparent ubiquity of the system in the animal and plant cells, this system is thought to serve an essential role for protein folding, function and possibly trafficking within the cytoplasm and nucleus.

We first tested this type ofR recombinase regulation in plants using SR1 recombinase fused to the rat GR LBD in the RG-construct (Fig. 1). GUS staining of leaves revealed that even without the ligand DEX some RG-derived transgenic lines carried leaves with a large number oftiny spots. This indicated that a low rate of spontaneous and premature loss of the Rs flanked DNA-segment was occurring and that the regulatory capacity of the LBD was less than could be hoped for. On the other hand, one of the earliest papers about ligand-regulated site-specific recombination reports approximately 1% background activity, i.e. in the absence of ligand the recombinase-LBD fusion displayed roughly 1% ofthe activity ofthe unmodified recombinase. Given sufficient time such a low-level activity might certainly explain the observed staining patterns.

The Southern blot analysis (Fig. 2) provides clear evidence that ligand-regulated site-specific recombination - in this case featuring R recombinase and rat GR LBD - is attainable in plants as well. It also illustrates the clear correlation between the variegated nature ofthe GUS-stained leaves (data not shown) and the presence of two different genomic fragments; one with and another without the recombinase-LBD fusion gene. In other words, without any further selection DEX-treated tissues tend to become chimaeric, i.e. consisting of a mixture of tissues in which site-specific recombination did or did not take place. To our knowledge this is the first demonstration ofa glucocorticoid-regulated recombinase in plants.

One major application of the ability to selectively remove an arbitrary DNA fragment from a stretch introduced previously via transformation would be the removal ofthe selectable marker following the regeneration of transgenic material. To this end, the marker would have to be inserted between the directly repeated recombination sites, preferably together with the recombinase-LBD gene. Following the transformation and regeneration of transgenic plants, the induction of recombinase activity would lead to excision of both the recombinase and the marker gene, leaving one intact recombination site and the remainder of the transferred DNA outside the region flanked by recombination sites. However, as was evident from the results of the work with the RG-transgenics, most of the treated tissues do not undergo complete recombination even in the least complicated situation, i.e. when only a single-copy insertion is present. In other words, following the DEX-treatment, cells that retain the marker-free cells will be surrounded by those that retained the marker and vice versa leading to the formation ofchimaeric tissue. One way to prevent growth of marker-containing cells and to obtain purely marker-free transgenics would be to employ a negative marker as well and to incorporate it on the segment that is eliminated by the recombinase. Several negative selectable markers functioning in plants have been described that confer dominant lethal phenotypes (Depicker *et al.,* 1988; Czako and An, 1991; Stougaard, 1993; Czako and Marton, 1994; Dotson *et al.,* 1996). Interestingly, one of these negative selectable markers - the *Escherichia coli* gene encoding cytosine deaminase *(codA) -* has also been shown to be suitable as a positive selectable marker rendering it bifunctional (Wei and Huber, 1996). Unfortunately, this system only works in mammalian cells and not in plant cells (unpublished observations).

The abundance of multifunctional enzymes in the animal and plant kingdoms suggested that a translational fusion of the coding regions ofa well-known positive and negative selectable marker might be a simple way of creating an artificial bifunctional marker gene. Thus, one could avoid having to supply a separate promoter and terminator for the negative selectable marker, which would have to be squeezed into the *Ac* element or between the recombination sites in a MAT-like transformation system together with the positive selectable marker and its own control sequences. To this end, we fused the coding region of the neomycin phosphotransferase II (*npt*II) gene to a cytosine deaminase gene (e.g. obtained from *Escherichia coli* although this is not necessary and it is even preferred to provide a more or less synthetic variant) and put the hybrid gene under the control of the enhanced CaMV 35S promoter. It is preferred to provide a hybrid marker wherein the nptII gene or functional fragment tehereofis the N-terminal enzyme instead of the cytosine deaminase.

The *nptII* gene has previously been used successfully in creating hybrid genes (Vaeck *et al.,* 1987; Barnes, 1990; Datla *et al.,* 1991). Similarly, we had previously utilised the cytosine deaminase as one of the two components ofa hybrid gene and shown it to confer 5-fluorocytosine (FC) sensitivity in plants (unpublished observations).

Transformation of potato with pMCODNPT yielded equal numbers of transformants irrespective of the antibiotic used for selection, which suggests that both markers performed alike and also that the *npt*II gene functioned will within the context of the fusion gene. The results presented in Table 2 demonstrate that the cytosine deaminase part of the fusion protein is also active.

In the RCNG construct that was built to test the performance of the bifunctional *codA-npt*II marker in a recombinase-LBD mediated marker removal system, both genes were present on the Rs-flanked DNA segment destined for elimination from the plant. The existence of completely GUS-positive transgenics obtained following transformation with the RG-construct already pointed to the possibility of premature loss of the Rs-flanked region at a very early stage following the transformation. Similar untimely, albeit much later, loss of the Rs-flanked DNA segment was also evident from the spots on the leaves of untreated RG- and RCNG-containing transgenic lines (data not shown). This suggests that sometimes control of the recombinase activity of the hybrid enzyme is insufficient even in the absence of the ligand. Nevertheless, the fact that RCNG-transgenic lines were readily obtained on media with kanamycin suggests that premature site-specific deletion, which would have removed the antibiotic resistance, may only happen occasionally. On the other hand, the presence of kanamycin in the regeneration medium following transformation with the RCNG-transformants essentially precluded the occurrence of completely GUS-positive shoots as observed following transformation with the RG-construct. Furthermore, transgenics with multicopy insertions - occurring quite regularly - would be less vulnerable assuming that complete removal of all copies at the same time is improbable. Finally, it is likely that the loss of the marker from the plant chromosome would also be less detrimental if it occurred in mitotically inactive cells, because the ensuing closed circle might still express the marker and confer antibiotic resistance (Ahmad and Golic, 1996).

Selection of marker-free strawberry plants was performed at an early stage following the transformation with RCNG, i.e. at the time when the first tiny shoots emerged. After an overnight DEX-treatment, the leaf discs were immediately transferred to FC-containing medium. However, this may not be the optimal treatment as the episomal closed circle excised from the genome still contains an active bifunctional marker that may be growth inhibitory at the least if not deleterious in the presence of FC! It would have been better to delay the transfer to FC-containing medium to allow at least one, but preferably two or three cell divisions. Then, truly marker-free cells, that also lost the closed circle with the marker that was excised from the chromosome, would have existed at the onset of FC-selection. Nevertheless, some marker-free cells were able to divide despite the toxin produced internally - at least initially - and by surrounding cells and were able to grow out into FC-resistant plants that lacked the marker.

PCR analysis was used to verify the excision of the marker and the recombinase. Clearly, most of the lines tested were marker-free. The presence of the predicted small fragment obtained by in the assays containing 35S and GUS primers, together with the absence of a fragment representing the *npt*II gene, indicates not only that recombination did take place but also that it was essentially complete.

Hence, it seems that LBD-mediated suppression of recombinase activity is generally sufficient to prevent high rates of premature excision thus allowing regeneration oftransgenic shoots under selective conditions. Nevertheless, it might be helpful to have an improved version of the LBD at one's disposal. In this way, it might be possible to reduce even further the frequency of untimely excisions. If the GUS-positive spots observed in our experiment are indeed due to high recombinase activity that could not be completely controlled, and if that high recombinase expression correlates positively with high expression of adjacent genes on the same T-DNA, then it can not be excluded that the RCNG-transgenics with the highest levels of expression of the gene-of-interest are already lost at an earlier stage of the transformation.

The circumstances for removing the marker following the transformation may become very complicated ifmultiple insertions are present either at the same locus or at different loci, and especially so if two or more inversely orientated T-DNAs are present at one locus. In the latter case, a very complex series of excisions and inversions may take place depending on which recombination sites interact and the outcome of the process will be totally unpredictable. If two or more T-DNAs are present in head-to-tail configuration, however, the final product of the recombinations may be indistinguishable from that derived from a transgenic with only one T-DNA per cell. Thus, one favourable side effect of the method could be that it might even lead to removal of undesirable directly repeated multiple copies of a T-DNA, keeping just one copy. With two single insertions at different loci, the undesirable consequence of the recombination process could be the removal of only one of the two markers and its accompanying recombinase gene. This could happen, for example, if only one of the two recombinase genes were actively described and if the inactive marker and recombinase gene would somehow escape excision.

Multiple copies ofa T-DNA integrated at different loci could be the cause of translocation of chromosome fragments (Qin *et al,* 1994; van Deursen *et al.,* 1995). When the two interacting recombination sites from different loci are situated in the same orientation with respect to the centromere, the translocation will be reciprocal and might well go unnoticed. In contrast, recombination between recombination sites from different loci oppositely oriented with respect to the centromeres would probably be detrimental due to chromosome loss. These translocations may also occur when a transformant, that has already gone through the process at least once, is transformed again with the same system. In all likelihood the T-DNA fragment with the recombination sites left after previous transformations will be located on other chromosomes than the latest insertion. Thus, recombination could take place between the single recombination site at one the previously transformed loci and one of the sites at the new locus. The chances of that actually happening depend on a number of factors like for example the proximity of the two loci, the concentration of the recombinase and the duration of the recombinase treatment. Spontaneous site-specific recombination was rare in the progeny from crosses between different transgenic tobacco lines carrying recombination sites on nonhomologous chromosomes (Qin *et al.,* 1994). It was not until selective pressure was applied that the frequency of recombination rose. Similarly, the estimated frequency of translocation between nonhomologous mouse chromosomes was approximately 1 in 1200-2400 cells expressing Cre recombinase (van Deursen *et al.,* 1995).

One way of reducing potential translocations in subsequent rounds of transformations and marker removal would be to use different recombination sites for each transformation (Sauer, 1996). A recombination site like loxP, Rs or FRT consists of two or more 12-13 bp inverted repeats separated by a 7-8 bp core at which the recombinase binds. Cleavage and strand exchange occur in the core sequence. Recombination is impaired between nonhomologous core sequences. Therefore, the core sequence determines the specificity of recombination. Variant recombination sites having a mutated core region can not recombine with the wild type site, but can recombine with each other. However, not all ofthe possible sites are capable of self recombination (Hoess *et al.,* 1986). For example, mutation of the central TpA dinucleotide in the loxP core abolishes recombination and multiple mutations become deleterious to the recombination of FRT sites when the high A+T content of their cores is decreased too much.

In our experiments described above no attempt was made to optimise the DEX treatment with regard to duration, concentration of DEX or the nature of the treated tissue. The duration of the treatment may affect the recombination efficiency and thus cause the formation ofchimaeric tissues if the tissue is not treated long enough. Alternatively, chimaerics may arise due to reintegration of the excised DNA into the genome. Due to the bimolecular nature of the reintegration, it is a less likely event than the unimolecular excision. In fact, if it happens at all it will presumably be immediately following the excision. At a later stage, the single excised molecule will probably have diffused away into the nucleus and is then unlikely to be recaptured into the genome. In transgenic mice it was demonstrated that such an episomal circular DNA fragment was lost at a rate of approximately 50% per month in resting cells (Rohlmann *et al.,* 1996). After the cell has divided, site-specific reintegration would be possible in only one of the two daughter cells provided the excised molecule and the recombinase survived the cell division. In summary, it is doubtful that reintegration might be influenced by the duration of the DEX treatment.

Of course, the concentration of DEX is another factor influencing the fraction of cells in a tissue that will respond to the treatment. So far, the published experiments with DEX in plants have been carried out at concentrations ranging between 0.1 and 30 µM to induce glucocorticoid-mediated transcription. However, the circumstances under which DEX was applied differed vastly from being present in the growth medium of seedlings in shaken liquid cultures to being sprayed onto the leaves. DEX was even supplied to the plants by watering the soil (Simon *et al.,* 1996). All of this suggests that plants readily take up DEX and that the nature of the tissue may not be relevant.

Site-specific recombination does not seem to require actively dividing cells, although such kinds of tissues were used in our experiments with potato axillary buds and strawberry calli. Experiments in mice and Drosophila involving Cre and FLP recombinase, respectively, demonstrate that recombination is achievable in tissues mainly consisting of resting cells (Kühn *et al.,* 1995; Ahmad and Golic, 1996). In fact, overnight treatment of fully-grown leaves from several potato RG- and RCNG-transgenics in liquid medium with DEX was sufficient to achieve strong GUS staining the next day. Actually, this method could be used to select recombination-proficient transgenic lines.

In the MAT system, premature recombinase activity lowers the transformation frequency and the low and uncontrolled recombinase activity does not provide more than a relatively slow harvest of marker-free transformants (Ebinuma *et al.,* 1996). In contrast, the system described in this paper provides control over recombinase activity and a clear-cut selection of marker-free transgenics. Nevertheless, improved negative markers and reduced recombinase background activity might boost the efficiency of this system. Ultimately, this would enable the development ofa universal system for removal of plant (and bacterial) selectable markers from the transgenic plant allowing multiple cycles of transformation and marker removal using the same protocol.

### REFERENCES

Ahmad K, Golic KG (1996) Somatic reversion of chromosomal position effects in Drosophila melanogaster. Genetics: 144: 657-670
Allefs JJ, Salentijn EM, Krens FA, Rouwendal GJA (1990) Optimization of nonradioactive Southern blot hybridization: single copy detection and reuse of blots. Nucleic Acids Res 18: 3099-3100
Aoyama T, Dong CH, Wu Y, Carabelli M, Sessa G, Ruberti I, Morelli G, Chua NH (1995) Ectopic expression of the Arabidopsis transcriptional activator Athb-1 alters leaf cell fate in tobacco. Plant Cell 7: 1773-1785
Aoyama T, Chua N-H (1997) A glucocorticoid-mediated transcriptional induction system in transgenic plants. Plant J 11: 605-612
Araki H, Nakanishi N, Evans BR, Matsuzaki H, Jayaram M, Oshima Y (1992) Site-specific recombinase, R, encoded by yeast plasmid pSR1. J Mol Biol 225: 25-7
Baulcombe DC, Saunders GR, Bevan MW, Mayo MA, Harrison BD (1986) Expression of biologically active viral satellite RNA from the nuclear genome of transformed plants. Nature 321: 446-449
Bevan M (1984) Binary Agrobacterium vectors for plant transformation. Nucl Acids Res 12: 8711-8721
Chambers SP, Prior SE, Bartsow DA, Minton NP (1988) The pMTL nic- cloning vectors. I. Improved pUC polylinker regions to facilitate the use of sonicated DNA for nucleotide sequencing. Gene 68: 139-149
Czako M, An G (1991) Expression of DNA coding for diphteria toxin chain A is toxic to plant cells. Plant Physiol 95: 687-692
Czako M, Marton L (1994) The herpes simplex virus thymidine kinase gene as a conditional negative-selection marker gene in Arabidopsis thaliana. Plant Physiol 104: 1067-1071
Dale EC, Ow DW (1991) Gene transfer with subsequent removal of the selection gene from the host genome. Proc Natl Acad Sci U S A 1991 88: 10558-10562
Depicker AG, Jacobs AM, Van Montagu MC (1988)A negative selection scheme for tobacco protoplast-derived cells expressing the T-DNA gene 2. Plant Cel Rep 7: 63-66
Dotson SB, Lanahan MB, Smith AG, Kishore GM (1996) A phosphonate monoester hydrolase from Burkholderia caryophilli PG2982 is useful as a conditional lethal gene in plants. Plant J 10: 383-392
Düring K, Porsch P, Fladung M, Lörz H (1993) Transgenic potato plants resistant to the phytopathogenic bacterium Erwinia carotovora. Plant J 3: 587-598
Ebinuma H, Sugita K, Matsunaga E, Yamakado M (1996) Vector and methods for introducing at least two genes into a plant. Patent WO96/15252
Ebinuma H, Sugita K, Matsunaga E, Yamakado M (1997a) Selection of marker-free transgenic plants using the isopentenyl transferase gene. Proc Natl Acad Sci USA 94: 2117-2121
Ebinuma H, Sugita K, Matsunaga E, Yamakado M, Komamine A (1997b) Principle of MAT vector system. Plant Biotechnology 14: 133-139
Edwards GA, Hepher A, Clerk SP, Boulter D (1991) Pea lectin is correctly processed, stable and active in leaves of transgenic potato plants. Plant Mol Biol 17: 89-100
Flavell RB, Dart E, Fuchs RL, Fraley RT (1992) Selectable marker genes: safe for plants? Bio/Technology 10: 141-144
Gleave Ap, Mitra DS, Mudge SR, Morris BAM (1999) Selectable marker-free transgenic plants without sexual crossing: transient expression of cre recombinase and use ofa conditional lethal dominant gene. Plant Mol Biol 40: 223-235
Goldsbrough AP, Lastrella CN, Yoder JI (1993) Transposition mediated re-positioning and subsequent elimination of marker genes from transgenic tomato. Bio/Technology 11: 1286-1292
Haasen D, Köhler C, Neuhaus G, Merkle T (1999) Nuclear export of proteins in plants: AtXPO1 is the export receptor for leucine-rich nuclear export signals in Arabidopsis thaliana. Plant J 20: 695-705
Haymes KM (1996). A DNA mini-prep method suitable for a plant breeding program. Plant Mol Biol Rep 14: 280-284
Heerklotz D, Döring P, Bonzelius F, Winkelhaus S, Nover L (2001) The balance of nuclear import and export determines the intracellular distribution and function of tomato heat stress transcription factor HsfA2. Mol Cell Biol 21: 1759-1768
Ho SN, Hunt HD, Horton RM, Pullen JK, Pease LR (1989) Site-directed mutagenesis by overlap extension using the polymerase chain reaction. Gene 77: 51-59
Hoess RH, Wierzbicki A, Abrem ski K (1986) The role of the loxP spacer region in P1 site-specific recombination. Nucleic Acids Res 14: 2287-2300
Hutchison KA, Dittmar KD, Pratt WB (1994) All of the factors required for assembly of the glucocorticoid receptor into a functional heterocomplex with heat shock protein 90 are preassociated in a self-sufficient protein folding structure, a "foldosome". J Biol Chem 1994 269:27894-27899
Jefferson RA (1987) Assaying chimeric genes in plants: the GUS gene fusion system. Plant Mol Biol Rep 5: 387-405
Jobling SA, Gehrke L (1987) Enhanced translation ofchimaeric messenger RNAs containing a plant viral untranslated leader sequence. Nature 325: 622-625
Kay R, Chan A, Daly M, McPherson J (1987) Duplication of CaMV 35S promoter sequences creates a strong enhancer for plant genes. Science 236: 1299-1302
Kudo N, Matsumori N, Taoka H, Fujiwara D, Schreiner EP, Wolff B, Yoshida M, Horinouchi S (199) Leptomycine B inactivates CRM1/exportin 1 by covalent modification at a cysteine residue in the central conserved region. Proc Natl Acad Sci USA 96: 9112-9116
Kühn R, Schwenk F Aguet M, Rajewsky K (1995) Inducible gene targeting in mice. Science 269: 1427-1429
Kurzer MS, Xu X (1997) Dietary phytoestrogens. Annu Rev Nutr 17: 353-381 Lyznik LA, Mitchell JC, Hirayama L, Hodges TK (1993) Activity of yeast FLP recombinase in maize and rice protoplasts. Nucleic Acids Res 1993 21: 969-975
Miesfeld R, Rusconi S, Godowski PJ, Maler BA, Okret S, Wikstroem A-C, Gustafsson J-A, Yamamoto KR (1986) Genetic complementation to a glucocorticoid receptor deficiency by expression of cloned receptor cDNA. Cell 46: 389-399
Murashige T, Skoog F (1962) A revised medium for rapid growth and bioassays with tobacco tissue cultures. Physiol Plant 15: 473-497
Nagel R, Elliott A, Masel A, Birch RG, Manners JM (1990) Electroporation of binary Ti plasm id vector into Agrobacterium tumefaciens and Agrobacterium rhizogenes. FEMS Microbiol Lett 67: 325-328
Oakes JV, Shewmaker CK, Stalker DM (1991) Production ofcyclodextrins, a novel carbohydrate, in the tubers oftransgenic potato plants. Bio/Technology 9: 982-986
Odell J, Caimi P, Sauer B, Russell S (1990) Site-directed recombination in the genome oftransgenic tobacco. Mol Gen Genet 1990 223: 369-378
Onouchi H, Yokoi K, Machida C, Matsuzaki H, Oshima Y, Matsuoka K, Nakamura K, Machida Y (1991) Operation of an efficient site-specific recombination system of Zygosaccharomyces rouxii in tobacco cells. Nucl Acids Res 23: 6373-6378
Onouchi H, Nishihama R, Kudo M, Machida Y, Machida C (1995) Visualization of site-specific recombination catalyzed by a recombinase from Zygosaccharomyces rouxii in Arabidopsis thaliana. Mol Gen Genet 247: 653-660
Perlak FJ, Fuchs RL, Dean DA, McPherson SA, Fischhoff DA (1991) Modification of the coding sequence enhances plant expression of insect control protein genes. Proc Natl Acad Sci USA 88: 3324-3328
Picard D, Salser SJ, Yamamoto KR (1988) A movable and regulable inactivation function within the steroid binding domain of the glucocorticoid receptor. Cell 1988 54:1073-1080
Pratt WB (1993) The role of heat shock proteins in regulating the function, folding, and trafficking of the glucocorticoid receptor. J Biol Chem 268: 21455-21458
Qin M, Bayley C, Stockton T, Ow DW (1994) Cre recombinase-mediated site-specific recombination between plant chromosomes. Proc Natl Acad Sci USA 91: 1706-1710
Rohlmann A, Gotthardt M, Willnow T, Hammer RE, Herz J (1996) Sustained somatic gene inactivation by viral transfer of Cre recombinase. Nature Biotechnol 14: 1562-1565
Rouwendal GJA, Mendes O, Wolbert E.J.H., de Boer A.D. (1997) Enhanced expression in tobacco of the gene encoding green fluorescent protein by modification of its codon usage. Plant Mol Biol 33: 989-999
Sarkar G, Sommer SS (1990) The "megaprimer" method of site-directed mutagenesis. BioTechniques 8: 404-407
Sauer B (1996) Multiplex Cre/lox recombination permits selective site-specific DNA targeting to both a natural and an engineered site in the yeast genome. Ncul Acids Res 24: 4608-4613
Schutte BC, Ranade K, Pruessner J, Dracopoli N (1991) Optimized conditions for cloning PCR products into an XcmI T-vector. Biotechniques 22: 40-42
Simon R, Igeño MI, Coupland G (1996) Activation of floral meristem identity genes in Arabidopsis. Nature 384: 59-62
Srivastava V, Anderson OD, Ow DW (1999) Single-copy transgenic wheat generated through the resolution of complex integration patterns. Proc Natl Acad Sci USA 96: 11117-11121
Stancato LF, Hutchison KA, Krishna P, Pratt WB (1996) Animal and plant lysates share a conserved chaperone system that assembles the glucocorticoid receptor into a functional heterocomplex with hsp90. Biochemistry 35: 554-561
Stougaard J (1993) Substrate-dependent negative selection in plants using a bacterial cytosine deaminase gene. Plant J 3: 755-761
Sugita K, Kasaharaq T, Matsunaga E, Ebinuma H (2000) A transformation vector for the production of marker-free transgenic plants containing a single copy transgene at high frequency. Plant J 22: 461-469
Van der Leij FR, Visser RG, Ponstein AS, Jacobsen E, Feenstra WJ (1991) Sequence of the structural gene for granule-bound starch synthase of potato (Solanum tuberosum L.) and evidence for a single point deletion in the amf allele. Mol Gen Genet 228: 240-248
Van Deursen J, Fornerod M, Van Rees B, Grosveld G (1995) Cre-mediated site-specific translocation between nonhomologous mouse chromosomes. Proc Natl Acad Sci USA 92: 7376-7380
Yamamoto KR (1985) Steroid receptor regulated transcription of specific genes and gene networks. Annu Rev Genet 19: 209-252
Yanisch-Perron C, Vieira J, Messing J (1985) Improved M13 cloning vectors and host strains: nucleotide sequences of M13mp18 and pUC19 vectors. Gene 33: 103-119
Yoshinaga SK, Yamamoto KR (1991) Signaling and regulation by a mammalian glucocorticoid receptor in Drosophila cells. Mol Endocrinol 5: 844-853 Zubko E, Scutt C, Meyer P (2000) Intrachromosomal recombination between attP regions as a tool to remove selectable marker genes from tobacco transgenes. Nature Biotech 18: 442-445
Zuo J, Niu QW, Chua NH (2000) An estrogen receptor-based transactivator XVE mediates highly inducible gene expression in transgenic plants. Plant J 24: 265-273

### Non-exhaustive list of markers

### Monofunctional, positive markers used in transgenic plants (selective agent between brackets):

□ *hpt* gene(hygromycin),
□ *npt* II gene (kanamycin),
□ *Bacillus subtilis protox* gene (oxyfluorfen),
□ Tn5-derived gene (bleomycin),
□ als gene (sulfonylurea),
□ *pat* or *bar* genes (phosphinothricin),
□ *Aspergillus bsd* gene (blasticidin),
□ Mouse or *Candida albicans dhfr* gene (methotrexate)
□ phosphomannose isomerase (PMI; allows growth on mannose as carbon source)
□ xylose isomerase (allows growth on xylose as carbon source)
□ *epsps* gene (glyphosate)
□ *dhps* gene (sulfonamide)
□ bacterial nitrilase gene (bromoxynil)
□ *psbA* gene (atrazine)
□ *spt* gene (streptomycin)
□ 2-DOG-6P phosphatase (2-deoxyglucose)

### Monofunctional, negative markers used in transgenic plants:

□ *E. coli* cytosine deaminase (enzyme converting fluorocytosine into fluorouracil)
□ *dhlA* gene from *Xanthobacter autotrophicus* GJ 10 encodes a dehalogenase which hydrolyzes dihalo- alkanes, such as 1, 2-dichloroethane (DCE), to a halogenated alcohol and an inorganic halide
□ HSV-1 Thymidine kinase (ganciclovir)
□ Barnase
□ *Streptomyces* cytochrome P450 mono-oxygenase gene (catalyses dealkylation ofa sulfonylurea compound, R7402, into cytotoxic sulfonylurea),
□ Nitrate reductase (enzyme converts chlorate to chlorite)
□ *IaaH* or *tms2* gene (enzyme converts auxin precursor to active auxin)
□ *pehA* gene (glyceryl glyphosphate to glyphosphate)
□ ricin (ribosome-inactivating protein)
□ diphteria toxin A

### Bifunctional markers used in plants:

□ ipt
□ GUS (cytokinin-glucuronide conjugate added to medium is converted to cytokinin in GUS-positive cells causing ipt-overexpression phenotype)

### Bifunctional markers used in animals:

□ HSVtk-*hpt* fusion
□ HSVtk-*npt* II fusion
□ HSVtk-*bsd* fusion

## Claims

1. A method for transforming a plant cell comprising providing said cell with a construct comprising a first nucleic acid encoding a first polypeptide with site-specific recombinase activity and a second nucleic acid encoding a polypeptide comprising a ligand binding domain (LBD) from a steroid receptor, who together encode a fusion polypeptide comprising site-specific recombinase activity and a functional ligand binding domain, and a third nucleic acid coding for a selectable marker, which can, after transformation, be excised by said recombinase.

2. A method according to claim 1 wherein said recombinase activity is ligand inducible.

3. A method according to any of claim lor 2 further comprising providing said cell with a nucleic acid encoding a desired functional trait.

4. A plant transformation vector comprising a construct having a first nucleic acid encoding a first polypeptide with site-specific recombinase activity and a second nucleic acid encoding a polypeptide comprising a ligand binding domain (LBD) from a steroid receptor which upon expression form a fusion polypeptide comprising site-specific recombinase activity and a functional ligand binding domain, and a third nucleic acid coding for a selectable marker, which can, after transformation, be excised by said recombinase.

5. A vector according to claim 4 wherein said first nucleic acid has a G+C content of at least 45 percent, preferably of at least 48 percent.

6. A vector according to any of claims 4-5 wherein said first nucleic acid encodes the R recombinase protein of *Zygosaccharomyces rouxii.*

7. A vector according to any of claims 4-6 wherein said selectable marker comprises a monofunctional positive marker, a monofunctional negative marker and/or a bifunctional marker.

8. A vector according to claim 7, wherein said monofunctional negative marker comprises cytosine deaminase activity.

9. A vector according to claim 7, wherein the bifunctional marker is a nucleic acid sequences which encodes a fusion protein comprising neomycine phosphotransferase or hygromycine phosphotransferase or phosphinotricin acetyl transferase with cytosine deaminase activity.

10. A method according to any of claims 1-3, wherein the ligand inducible site-specific recombinase activity and preferably the selectable marker and the desired functional trait are provided to a plant cell by introduction of a vector according to any of claims 4-9.

11. A transformed plant cell comprising a vector according to any of claims 4-9.

12. A method for depriving a plant or plant cell according to claim 11 of its inducible recombinase activity and also its selectable marker by comprising inducing said activity posttranslationally.

13. A method according to claim 12 wherein inducing said activity involves application of a steroid, preferably dexamethasone.

## Patentansprüche

1. Verfahren zur Transformation einer Pflanzenzelle, bei dem man die Zelle mit einem Konstrukt ausstattet, das eine erste Nukleinsäure umfasst, welche für ein erstes Polypeptid mit einer ortsspezifischen Rekombinase-Aktivität kodiert, und mit einer zweiten Nukleinsäure, die für ein Polypeptid kodiert, welches eine Liganden-Bindungsdomäne (LBD) eines Steroid-Rezeptors umfasst, welche zusammen für ein Fusionspolypeptid kodieren, das eine ortsspezifische Rekombinase-Aktivität und eine funktionelle Liganden-Bindungsdomäne umfasst, und mit einer dritten Nukleinsäure, die für einen selektierbaren Marker kodiert, welcher nach der Transformation durch die Rekombinase entfernt werden kann.

2. Verfahren nach Anspruch 1, bei dem die Rekombinase-Aktivität Liganden-induzierbar ist.

3. Verfahren nach einem der Ansprüche 1 oder 2, bei dem die Zelle ferner mit einer Nukleinsäure, die für ein gewünschtes funktionelles Merkmal kodiert, ausgestattet wird.

4. Pflanzen-Transformationsvektor, der ein Konstrukt mit einer ersten Nukleinsäure, die für ein erstes Polypeptid mit einer ortsspezifischen Rekombinase-Aktivität kodiert, und einer zweiten Nukleinsäure, die für ein Polypeptid, welches eine Liganden-Bindungsdomäne (LBD) eines Steroid-Rezeptors umfasst, kodiert, wobei diese bei der Expression ein Fusionspolypeptid bilden, das eine ortsspezifische Rekombinase-Aktivität und eine funktionelle Liganden-Bindungsdomäne umfasst, und mit einer dritten Nukleinsäure, die für einen selektierbaren Marker kodiert, welcher nach der Transformation durch die Rekombinase entfernt werden kann, umfasst.

5. Vektor nach Anspruch 4, bei dem die erste Nukleinsäure einen G+C-Gehalt von mindestens 45% aufweist, vorzugsweise von mindestens 48%.

6. Vektor nach einem der Ansprüche 4-5, bei dem die erste Nukleinsäure für das Rekombinase-Protein R von *Zygosaccharomyces rouxii* kodiert.

7. Vektor nach einem der Ansprüche 4-6, bei dem der selektierbare Marker einen monofunktionalen positiven Marker, einen monofunktionalen negativen Marker und/oder einen bifunktionalen Marker umfasst.

8. Vektor nach Anspruch 7, bei dem der monofunktionale negative Marker eine Cystosin-Deaminase-Aktivität umfasst.

9. Vektor nach Anspruch 7, bei dem der bifunktionale Marker eine Nukleinsäuresequenz ist, die für ein Fusionsprotein kodiert, welches eine Neomycin-Phosphotransferase oder eine Hygromycin-Phosphotransferase oder eine Phosphinotricin-Acetyl-Transferase mit Cystosin-Deaminase-Aktivität umfasst.

10. Verfahren nach einem der Ansprüche 1-3, bei dem eine Pflanzenzelle mit der Liganden-induzierbaren, ortsspezifischen Rekombinase-Aktivität und vorzugsweise mit dem selektierbaren Marker sowie mit dem gewünschten funktionalen Merkmal ausgestattet wird, indem man einen Vektor nach einem der Ansprüche 4-9 einbringt.

11. Transformierte Pflanzenzelle, die einen Vektor nach einem der Ansprüche 4-9 umfasst.

12. Verfahren, um einer Pflanze oder einer Pflanzenzelle nach Anspruch 11 seine induzierbare Rekombinase-Aktivität und auch den selektierbaren Marker zu entziehen, bei dem man die Aktivität posttranslational induziert.

13. Verfahren nach Anspruch 12, wobei das Induzieren der Aktivität die Anwendung eines Steroids, vorzugsweise die Anwendung von Dexamethason umfasst.

## Revendications

1. Procédé de transformation d'une cellule végétale comprenant la mise à disposition de ladite cellule avec un montage comprenant un premier acide nucléique codant un premier polypeptide ayant une activité recombinase spécifique du site et un second acide nucléique codant un polypeptide comprenant un domaine de liaison de ligand (LBD) provenant d'un récepteur de stéroïde, lesquels codent ensemble un polypeptide de fusion comprenant une activité recombinase spécifique du site et un domaine fonctionnel de liaison de ligand, et un troisième acide nucléique codant un marqueur sélectionnable qui peut, après transformation, être excisé par ladite recombinase.

2. Procédé selon la revendication 1 où ladite activité recombinase est inductible par un ligand.

3. Procédé selon l'une quelconque des revendications 1 et 2 comprenant en outre la mise à disposition de ladite cellule avec un acide nucléique codant une caractéristique fonctionnelle désirée.

4. Vecteur de transformation végétale comprenant un montage ayant un premier acide nucléique codant un premier polypeptide ayant une activité recombinase spécifique du site et un second acide nucléique codant un polypeptide comprenant un domaine de liaison de ligand (LBD) provenant d'un récepteur de stéroïde, lesquels, après expression, forment un polypeptide de fusion comprenant une activité recombinase spécifique du site et un domaine fonctionnel de liaison de ligand, et un troisième acide nucléique codant un marqueur sélectionnable qui peut, après transformation, être excisé par ladite recombinase.

5. Vecteur selon la revendication 4 où ledit premier acide nucléique a une teneur en G+C d'au moins 45 %, de préférence d'au moins 48 %.

6. Vecteur selon l'une quelconque des revendications 4 et 5 où ledit premier acide nucléique code la protéine recombinase R de *Zygosaccharomyces rouxii.*

7. Vecteur selon l'une quelconque des revendications 4 à 6 où ledit marqueur sélectionnable comprend un marqueur positif monofonctionnel, un marqueur négatif monofonctionnel et/ou un marqueur bifonctionnel.

8. Vecteur selon la revendication 7, où ledit marqueur négatif monofonctionnel comprend une activité cytosine désaminase.

9. Vecteur selon la revendication 7, où le marqueur bifonctionnel est une séquence d'acides nucléiques qui code une protéine de fusion comprenant la néomycine phosphotransférase ou l'hygromycine phosphotransférase ou la phosphinotricine acétyltransférase ayant une activité cytosine désaminase.

10. Procédé selon l'une quelconque des revendications 1 à 3, où l'activité recombinasse spécifique du site inductible par un ligand et de préférence le marqueur sélectionnable et la caractéristique fonctionnelle désirée sont apportés à une cellule végétale par introduction d'un vecteur selon l'une quelconque des revendications 4 à 9.

11. Cellule végétale transformée comprenant un vecteur selon l'une quelconque des revendications 4 à 9.

12. Procédé de privation d'une plante ou d'une cellule végétale selon la revendication 11 de son activité recombinase inducible est également de son marqueur sélectionnable par le fait qu'il comprend une induction post-traductionnelle de ladite activité.

13. Procédé selon la revendication 12 où induction de ladite activité implique l'application d'un stéroïde, de préférence la dexaméthasone.
